# EUROPEAN PATENT APPLICATION

(11) **EP 2 090 572 A1**
(43) Date of publication of application: **19.08.2009**
(21) Application number: 07830372.4
(22) Date of filing: 23.10.2007
(51) Int. Cl.: C07D 303/48, C07D 301/12

(54) **PROCESS FOR PRODUCTION OF HEXAFLUOROPROPYLENE OXIDE**

(30) Priority: 24.10.2006 JP 2006288467
(71) Applicant: Daikin Industries, Ltd., Osaka 530-8323 (JP)
(72) Inventor: NAKAYA, Hideki, Settsu-shi Osaka 5668585 (JP); ASANO, Michio, Settsu-shi Osaka 5668585 (JP); SAITOU, Hideya, Settsu-shi Osaka 5668585 (JP)
(74) Representative: HOFFMANN EITLE
(86) International application number: PCT/JP2007/070638
(87) International publication number: WO 2008/050760

(57) **Abstract**

There is provided a process for producing hexafluoropropylene oxide which is novel and capable of achieving a higher yield. An organic phase comprising hexafluoropropylene (HFP) in an organic solvent and an aqueous phase comprising an oxygen-containing oxidizing agent in water are supplied to a small space (or microspace), preferably together with a phase transfer catalyst. The organic phase and the aqueous phase are contacted with each other in the small space, thereby reacting hexafluoropropylene (HFP) with the oxygen-containing oxidizing agent, preferably by an action of the phase transfer catalyst to give hexafluoropropylene oxide (HFPO). After the reaction, the organic phase and the aqueous phase are taken out from the small space to obtain an organic phase comprising the hexafluoropropylene oxide (HFPO).

## Description

### Technical Field

The present invention relates to a process for producing hexafluoropropylene oxide, and more particularly to a process for producing hexafluoropropylene oxide by oxidation of hexafluoropropylene.

### Background Art

Hexafluoropropylene oxide is an important compound in the production of fluorine-containing compounds, since it is used, for example, as a raw material for perfluorovinylether. An oligomer of the hexafluoropropylene oxide is utilized as a lubricating oil or a heating medium.

As a process for producing hexafluoropropylene oxide (hereinafter also referred to as HFPO), various process for producing HFPO by oxidation of hexafluoropropylene (hereinafter also referred to as HFP) have hitherto been developed.

For example, there is a high-temperature vapor-phase reaction process in which HFP is oxidized with oxygen in the presence of a catalyst such as SiO₂, CuO or BaO or under catalyst-free conditions to obtain HFPO. There is other process to obtain HFPO from HFP by using ultraviolet rays. In addition, there is a low-temperature liquid-phase reaction process in which HFP is oxidized with potassium permanganate in an HF solvent to obtain HFPO, and a low-temperature liquid-phase reaction process in which HFP is oxidized with hydrogen peroxide in a methanol solvent to obtain HFPO.

There is also known a liquid-phase reaction process in which HFP is oxidized with oxygen in a solvent such as 1,1,2-trichloro-1,2,2-trifluoroethane (CFC-113) to obtain HFPO (refer to Patent Citation 1). According to this process, a higher yield (about 60%) can be industrially achieved when compared with the conventional processes described above.

For the purpose of achieving a higher yield, there has also recently been proposed a process in which HFP is oxidized with oxygen in the presence of an aldehyde having an electron-withdrawing group, which is represented by the general formula: RCHO (wherein R is a monovalent electron-withdrawing hydrocarbon group) (refer to Patent Citation 2).

Patent Citation 1: Japanese Patent Publication for Opposition (Kokoku) No. 45-11683
Patent Citation 2: Japanese Patent Laid-open Publication (Kokai) No. 2006-83152
Patent Citation 3: Japanese Patent Publication for Opposition (Kokoku) No. 64-11021
Patent Citation 4: Japanese Patent Publication for Opposition (Kokoku) No. 3-75546
Patent Citation 5: Japanese Patent No. 3785652
Non Patent Citation 1: Hideho Okamoto, "Possibility of application of the micro reactor to green process", FARUMASHIA, 2005, The Pharmaceutical Society of Japan, Vol.41, No.7, p664

### Disclosure of Invention

### Technical Problem

However, the yield of HFPO by the conventional processes described above is not necessarily satisfactory, and a further improvement is required.

An object of the present invention is to provide a process for producing hexafluoropropylene oxide, which is novel and capable of achieving a higher yield of HFPO.

### Technical Solution

In the meantime, there has been proposed as a process for producing hexafluoropropylene oxide (HFPO), a liquid phase reaction process in which HFP is oxidized with a hypochlorite in a two-phase system of an aqueous phase and an organic phase in the presence of a quaternary ammonium salt or the like to obtain HFPO (refer to Patent Citation 3 and Patent Citation 4). The present inventors have intensively studied taking notice of this process above all, and thus the present invention has been completed.

According to one aspect of the present invention, there is provided a process for producing hexafluoropropylene oxide, which comprises passing an organic phase comprising hexafluoropropylene and an aqueous phase comprising an oxygen-containing oxidizing agent through a small space (or microspace) in contact with each other, thereby reacting hexafluoropropylene with the oxygen-containing oxidizing agent to obtain hexafluoropropylene oxide.

The test results of the present inventors revealed that the present invention enables remarkably higher yield than that of any conventional processes, although it depends on the reaction conditions. While the present invention is not intended to be bound by any specific theory, the reason is considered to be as follows. An oxidation reaction for obtaining hexafluoropropylene oxide (HFPO) from hexafluoropropylene (HFP) is an exothermic reaction. The reaction proceeding in the small space makes it possible to attain efficient heat removal and strict temperature control, and thus side reactions can be suppressed and the selectivity of HFPO can be improved. Further, the reaction for obtaining HFPO from HFP involves diffusion. The reaction proceeding in the small space makes it possible to carry out the reaction sufficiently within a shorter reaction time. Furthermore, the organic phase and the aqueous phase contacting with each other in the small space makes it possible to increase a liquid-liquid interfacial area, and thus to increase the reaction rate. Therefore, the attainment of sufficient heat removal and strict temperature control prevents the selectivity from decreasing while the reaction rate increases, and the shortened reaction time (or residence time) makes it possible that produced HFPO can be instantly discharged out of the reaction system (the small space) to prevent a further reaction (over reaction) of the product, and as a result of these, the reaction can be realized with a high selectivity and a high conversion rate, and thus the yield of HFPO can be improved.

In the present invention, the term "small space" (or microspace) means a space having a width of a passage of 3 cm or less, preferably not less than 1 µm and not more than 1 cm (micro-order or milli-order), through which a fluid for the reaction flows (in the present invention, the fluid includes a liquid comprising an aqueous phase and an organic phase and a vapor phase which may optionally exist), and the width of the passage means a minimum distance between opposing wall surfaces of the passage. Such the "small space" may be each passage (or channel) of a reactor or a mixer known as a "microreactor" or a "micromixer" in the fields of pharmaceutical, synthetic chemistry and the like (for example, refer to Non Patent Citation 1).

In a preferred aspect of the present invention, an organic phase comprising hexafluoropropylene and an aqueous phase comprising an oxygen-containing oxidizing agent are contacted with each other in the presence of a phase transfer catalyst, thereby reacting hexafluoropropylene with the oxygen-containing oxidizing agent by an action of the phase transfer catalyst. Thus, it becomes possible to cause the reaction more efficiently.

In one aspect of the present invention, the small space is maintained at a temperature of about -40 to 100°C and a pressure of about 0.1 to 20 MPa, appropriately. The temperature more than 100°C and/or the pressure more than 20 MPa is not preferable since the situation may approach the exploding conditions of HFPO. On the other hand, the temperature less than -40°C and/or the pressure less than 0.1 MPa is not preferable since water of the aqueous phase becomes ice. When the temperature and the pressure are adjusted within the above ranges, it becomes possible to obtain the yield of about 70% or more, and preferably about 90% or more, although it depends on other conditions such as the use and kind of a phase transfer catalyst.

In a preferred embodiment of the present invention, preliminary adjustment is carried out so as to suitably maintain a temperature of about -40 to 100°C and a pressure of about 0.1 to 20 MPa, and more preferably the temperature and pressure conditions at which hexafluoropropylene (HFP) is substantially in a liquid state, before the organic phase is supplied to the small space. Since hexafluoropropylene is a gas at a normal temperature under a normal pressure (boiling point: - 29.4°C), the preliminary adjustment conducted upon supplying the organic phase to the small space makes more amount of HFP dissolved in the organic phase, thus enabling the liquid phase reaction to efficiently proceed.

The reaction time in the small space can be from about 0.01 to 1,000 seconds. Such the reaction time is very short when compared with the reaction time in a conventional reaction space volume which has been used generally.

The "phase transfer catalyst" to be used in a preferred embodiment of the present invention may be a substance which can move between an aqueous phase and an organic phase in any form because of its affinity with both the aqueous phase and the organic phase and promote the reaction by transporting a nucleophilic portion of an oxygen-containing oxidizing agent, which is mostly distributed in the aqueous phase, to the organic phase. For example, a quaternary ammonium salt(s) is preferably used as the phase transfer catalyst, and has advantages of excellent affinity with both the organic phase and the aqueous phase, of various kinds of commercially available reagents, and of a relatively low price.

The "oxygen-containing oxidizing agent" to be used in the present invention may be an oxidizing agent which contains oxygen atoms and can oxidize HFP into HFPO. For example, a hypochlorite(s) is preferably used as the oxygen-containing oxidizing agent, and has an advantage that hypochlorous acid ions are produced under the reaction conditions and are converted into chlorine ions by reacting with HFP to form a salt having no oxidizing effect.

### Advantageous Effects

The present invention provides a process for producing hexafluoropropylene oxide, which can achieve remarkably higher yield than that of any conventional process.

### Brief Description of Drawings

Fig. 1 is a schematic view of an apparatus used to produce HFPO in Example 1 of the present invention.
Fig. 2 is a reaction scheme in Example 1 of the present invention.
Fig. 3 is a schematic view of an apparatus used to produce HFPO in Example 2 of the present invention.

### Explanation of Reference

1 HFP tank
3 Organic solvent bath (Organic solvent comprises a phase transfer catalyst)
5, 9, 15, 19, 23 Line
7, 17 Syringe pump
7a, 17a Pump room
7b Cooling jacket
13 Aqueous solution bath (Aqueous solution comprises an oxygen-containing oxidizing agent)
21 Capillary
21a Heating jacket
21b Connector
25 Back pressure valve
27 Recovery bath
31 Micromixer
31a Heating jacket

### Best Mode for Carrying Out the Invention

The process for producing hexafluoropropylene in one embodiment of the present invention is described in detail below.

Firstly, an aqueous phase comprising an oxygen-containing oxidizing agent and an organic phase comprising hexafluoropropylene (HFP) are respectively prepared, and also a phase transfer catalyst is prepared.

As the oxygen-containing oxidizing agent, for example, hypochlorite, chlorite, chlorate, perchlorate, ozone water, hydrogen peroxide water or the like can be used. Among them, hypochlorite is preferred since hypochlorous acid ions are produced under' the reaction conditions and are converted into chlorine ions by reacting with HFP to form a salt having no oxidizing effect. Hypochlorite contains an alkali metal salt and an alkali earth metal salt. Among them, a sodium salt is more preferred since it is industrially mass-produced for applications such as bleaching agents and sterilizers and thus it is available at a low price. The oxygen-containing oxidizing agent may be used in combination with an alkali. Alkalifying by adding an alkali such as sodium hydroxide or potassium oxide can prevent the decomposition of the oxidizing agent which would be caused by an acid as the reaction proceeds.

For a solvent (an aqueous solvent) of the aqueous phase, an aqueous substance capable of dissolving the oxygen-containing oxidizing agent therein can be used, and usually water is used.

The concentration of the oxygen-containing oxidizing agent in the aqueous phase is, for example, from about 1 to 20% by weight, and preferably from about 5 to 15% by weight, when supplied to the small space (or at the beginning of the reaction).

On the other hand, for an organic solvent of the organic phase, an inert solvent which is substantially immiscible or hardly miscible in the aqueous phase can be used. Examples of the organic solvent include aliphatic hydrocarbons, alicyclic hydrocarbons, aromatic hydrocarbons, chlorinated hydrocarbons, fluorinated hydrocarbons (CFC, HCFC, HFC) and perfluoropolyether. In order to dissolve HFP, halogen-containing compounds such as chlorinated hydrocarbons, fluorinated hydrocarbons, perfluoropolyether, and a mixture thereof are preferably used. Compounds having a fluorous property, e.g. fluorinated hydrocarbons (chlorofluorocarbon (CFC), hydrochlorofluorocarbon (HCFC), hydrofluorocarbon (HFC)) and perfluoropolyether are more preferred.
Aliphatic hydrocarbons, alicyclic hydrocarbons and aromatic hydrocarbons are not particularly limited, but preferably hexane, heptane, isoheptane, octane, isooctane, methylcyclopentane, cyclohexane, methylcyclohexane and toluene.
Examples of CFC include 1,1,2-trichloro-1,2,2-trifluoroethane (CFC-113) and so on.
Examples of HCFC include difluorochloromethane (HCFC-22), 1,1-dichloro-1-fluoroethane (HCFC-141b), 1,1-dichloro-2,2,2-trifluoroethane (HCFC-123), 1-chloro-1,1-difluoroethane (HCFC-142b), dichloropentafluoropropane (HCFC-225) and so on.
Examples of HFC include fluorinated aliphatic hydrocarbons represented by the general formula: CₓF_{y}H_{2x+2-y} (wherein x and y represents an integer satisfying the relations of 4 ≤ x ≤ 6 and 6 ≤ y ≤ 12), and specific examples thereof include compounds represented by C₄F₅H₅, C₄F₆H₄, C₄F₈H₂, C₅F₇H₅, C₅F₈H₄, C₅F₉H₃, C₅F₁₀H₂, C₆F₉H₅ and C₆F₁₂H₂, and are preferably 1,1,1,3,3-pentafluorobutane (HFC-365), 1,1,2,3,4,4-hexafluorobutane (HCF₂CFHCFHCF₂H), 1,1,1,2,2,3,3,4-octafluorobutane (CF₃CF₂CF₃CH₂F), 1,1,2,2,3,3,4-heptafluoropentane (HCF₂(CF₂)₂CFHCH₃), 1,1,2,3,3,4,5,5-octafluoropentane (HCF₂CFHCF₂CFHCF₂H), 1,1,2,2,3,3,4,4,5-nonafluoropentane (HCF₂(CF₂)₃CH₂F), 1, 1, 1, 2, 3, 3, 4, 4, 5, 5-decafluoropentane (CF₃CF(CHF₂)CF₂CF₂H), 1,1,1,2,2,3,3,4,4-nonafluorohexane (CF₃(CF₂)₃CH₂CH₃), 1,1,2,2,3,3,4,4,5,5,6,6-dodecafluorohexane (HCF₂(CF₂)₄CF₂H), 2-trifluoromethyl-1,1,1,3,4,4,5,5,5-nonalfluoropentane (CF₃)₂CHCFHCF₂CF₃ and so on.
Among these fluorinated hydrocarbons, HFC is preferred, especially 1,1,1,3,3-pentafluorobutane is preferred.
Examples of perfluoropolyether include compounds represented by the following general formula (I). The molecular weight of the compound represented by the general formula (I) is preferably from about 100 to 100,000, more preferably from about 250 to 50,000, and still more preferably from about 500 to 10,000.

R₁-(CFR₃-CF₂-CF₂-O)ₗ-(CFR₄-CF₂-O)ₘ-(CFR₅-O)ₙ-R₂ (I)

(In the formula, R₁, R₂, R₃, R₄ and R₅ each independently represents a fluorine atom or a perfluoroalkyl group; 1, m and n each independently represents 0 or a natural number, and at least one of l, m and n is not 0.)
As the perfluoropolyether, there may be used a compound represented by the following general formula (II) and a compound represented by the following general formula (III). 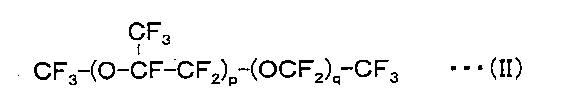 (In the formula, p and q each independently represents a natural number.)

F-(CF₂-CF₂-CF₂-O)ᵣ-CF₂CF₃ (III)

(In the formula, r represents a natural number.)

The hexafluoropropylene (HFP) as a reaction material may be obtained from tetrafluoroethylene.

The solubility of HFP in an organic solvent can depend on the temperature and pressure conditions, although it varies depending on the kind of the organic solvent to be used. Before the organic phase comprising HFP is supplied to the small space, such the organic phase (or HFP and an organic solvent coexisting with each other) is preferably subjected to substantially the same as or similar to the temperature and pressure conditions in the small space (this is also referred to as preliminary adjustment in this specification). For example, the organic phase can be preliminarily maintained at a temperature of about -40 to 100°C, preferably -10 to 50°C, and a pressure of about 0.1 to 20 MPa, preferably about 0.2 to 5 MPa, appropriately. The preliminary adjustment conditions are preferably adjusted to temperature and pressure conditions at which hexafluoropropylene is substantially in a liquid state. In order to allow the liquid phase reaction to efficiently proceed, it is more preferred to dissolve HFP as a reaction material in an organic phase in the amount as large as possible. However, since HFP is a gas at a normal temperature under a normal pressure (boiling point: -29.4°C), upon supplying the organic phase to the small space, the organic phase is preferably subjected to the temperature and pressure conditions at which HFP is substantially in a liquid state, so as to dissolve a larger amount, preferably substantially the entire amount of HFP in the organic phase. As described in detail hereinafter, since the reaction time (residence time) in the small space is very short and redistribution of HFP from the organic phase to the gas phase during the reaction is actually negligible, the temperature and pressure conditions for the preliminary adjustment may be different from those in the small space to which the organic phase is to be supplied.

The concentration of HFP in the organic phase is, for example, from about 0.5 to 100% by weight, preferably from about 1 to 50% by weight, and more preferably from about 2 to 20% by weight, when supplied to the small space (or at the beginning of the reaction).

As the phase transfer catalyst, for example, a quaternary ammonium salt, a quaternary phosphonium salt and a macrocyclic ether can be used. Among them, the quaternary ammonium salt is preferred since it has excellent affinity with both the organic phase and the aqueous phase and includes various commercially available reagents, and is available at a relatively low price.

The quaternary ammonium salt is represented by the formula shown below (wherein R1, R2, R3 and R4 represent a hydrocarbon group, for example, an alkyl group, and X⁻ represents an anion). The kind and carbon number in a carbon chain of the hydrocarbon groups (R1, R2, R3 and R4) can be arbitrary selected, and also the kind of anion (X⁻) can be arbitrary selected. 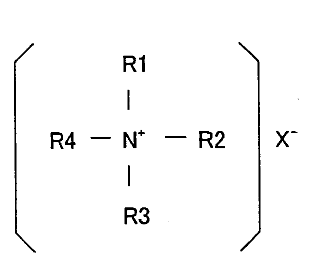 This selection can be appropriately made depending on the kind and amount (or concentration) of the oxygen-containing oxidizing agent and the kind and amount of the solvent which are to be used in the reaction system, the temperature and pressure of the reaction, and so on. As the quaternary ammonium salt, for example, tri-n-octylmethylammonium chloride (TOMAC), tetrabutylammonium hydrogen sulfate (TBAS) and tetrabutylammonium bromide (TBAB) can be used. In the reaction of the present invention, a quaternary ammonium salt showing high distribution in the organic phase comprising HFP is preferred, especially TOMAC is preferred.

The quaternary phosphonium salt is represented, for example, by the formula shown below (wherein R5, R6, R7 and R8 represent a hydrocarbon group, for example, an alkyl group, and Y⁻ represents an anion). The kind and carbon number in a carbon chain of the hydrocarbon groups (R5, R6, R7 and R8) can be arbitrary selected, and also the kind of anion (Y⁻) can be arbitrary selected. 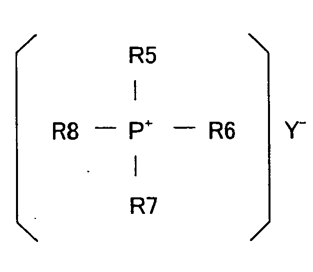 This selection can also be appropriately made depending on the kind and amount (or concentration) of the oxygen-containing oxidizing agent and the kind and amount of the solvent which are to be used in the reaction system, the temperature and pressure of the reaction, and so on. As the quaternary phosphonium salt, for example, tetra-n-butylphosphonium bromide, n-amyltriphenylphosphonium bromide and benzyltriphenylphosphonium chloride can be used, and tetra-n-butylphosphonium bromide is particularly preferred.

As long as the phase transfer catalyst is present when the aqueous phase and the organic phase are contacted with each other, it may be supplied to the small space in any form, but can be usually supplied in a state of being added to the aqueous phase or the organic phase. It is possible to judge either the aqueous phase or the organic phase shall incorporate the phase transfer catalyst, for example, based on intensity of either of lipophilicity or water solubility of a quaternary ammonium salt or a quaternary phosphonium salt. When the phase transfer catalyst is rather lipophilic, it is preferably added to the organic phase.

The concentration of the phase transfer catalyst in the aqueous phase or the organic phase is, for example, from about 0.5 to 20% by weight, and preferably from about 1 to 10% by weight, when supplied to the small space (or at the beginning of the reaction).

Next, the aqueous phase comprising an oxygen-containing oxidizing agent and the organic phase comprising HFP thus prepared are supplied to the small space, together with the phase transfer catalyst.

The small space may have a width of a passage, through which a fluid for the reaction flows (the fluid is comprised of the aqueous phase and the organic phase, and a vapor phase which may optionally exist), of 3 cm or less. For example, the width of the passage is from about 1 µm to 1 cm, and preferably from about 10 to 5,000 µm. As long as the width of the passage is within the above range, there is no particular limitation on the length of the passage and the cross-sectional area. The cross-sectional area of the passage can be, for example, from about 3.1 × 10⁻⁶ to 7.9 × 10⁻¹ cm². For example, a reactor (or a reaction tube) having at least one small space having an equivalent diameter of 20 µm to 2,000 µm, or a so-called "microreactor" or "micromixer" can be used.

The aqueous phase comprising an oxygen-containing oxidizing agent and the organic phase comprising hexafluoropropylene (HFP) flow in the small space, together with the phase transfer catalyst, to contact with each other. In this period, HFP is reacted with the oxygen-containing oxidizing agent in the presence of the phase transfer catalyst to give hexafluoropropylene oxide (HFPO).

Although there is no particular limitation on the contact between the organic phase and the aqueous phase in the small space, the contact is preferably carried out in a state of laminar flow. Whether the state is laminar flow can be determined based on Reynolds number, although it depends on the structure of an apparatus to be used.

The temperature and pressure in the small space are not particularly limited as long as the reaction for obtaining HFPO from HFP proceeds, and can be appropriately maintained at a temperature of about -40 to 100°C, preferably from about -10 to 50°C, and a pressure of about 0.1 to 20 MPa, preferably from about 0.2 to 5 MPa.

A ratio of volumes of the organic phase/the aqueous phase in the small space (or a ratio of supply flow rates of the organic phase/the aqueous phase) can be appropriately set according to a specific situation and is, for example, from about 0.1 to 10, and preferably from about 0.2 to 5.

The reaction time (or residence time) in the small space may be a shorter time than those of the conventional processes and is, for example, from about 0.01 to 1,000 seconds, particularly from about 0.01 to 100 seconds, and more particularly from about 0.01 to 50 seconds.

The organic phase and the aqueous phase after the reaction are taken out of the small space in any form, for example, in a mixed state or a separated state. Since HFPO is distributed in the organic phase, HFPO produced by the reaction can be recovered from the organic phase after the reaction. Since HFPO is gasified by depressurization, HFPO can be easily recovered from the organic phase.

The organic phase after the reaction may be optionally subjected to a post-treatment to remove unnecessary substances, for example, an unreacted HFP, a side reaction product(s) and the solvent.
After the present embodiment, known methods such as distillation, extraction, column chromatography, membrane separation and recrystallization may be used so as to purify the reaction mixture. Among these methods, distillation is used industrially and widely as a general separation operation. However, the unreacted HFP and HFPO as the objective product, which mainly make up the reaction mixture, have boiling points of -29.4°C and -27.4°C respectively, and due to closeness of the boiling points, it is difficult to separate them from each other by a distillation operation. Then, extraction distillation is preferably carried out so as to separate HFP from HFPO to obtain high-purity HFPO (refer to Patent Citation 5). HFP separated by extraction distillation may be reused as a raw material.
In the case of the extraction distillation, the solvent to be used in the organic phase is preferably a solvent which can be utilized as an extraction distillation solvent. The effectiveness of the extraction distillation solvent can be evaluated by the relative volatility of HFPO to HFP. The relative volatility can be measured by a method known in this technical field or a method described in Patent Citation 5. The relative volatility of HFPO to HFP is more than 1, and in general it is preferably 1.1 or more.
It is possible to use, as the solvent (which also serves as an extraction distillation solvent), a hydrogencontaining halogenated hydrocarbon represented by the following general formula (IV).

CₙHₐCl_{b}F_{c} (IV)

(In the formula, n represents an integer of 2 to 6; a represents an integer satisfying the relation of 1 ≤ a ≤ n+1; b represents an integer satisfying the relation of 1 ≤ b ≤ 2n; c represents an integer satisfying the relation of 1 ≤ c ≤ 2n; and a+b+c = 2n+2.)
More specifically, 1,1-dichloro-1-fluoroethane (HCFC-141b), 2,2-dichloro-1,1,1-trifluoroethane (HCFC-123), 1,2-dichloro-1,1,2-trifluoroethane (HCFC-123a), 3,3-dichloro-1,1,1,2,2-pentafluoropropane (HCFC-225ca), 1,3-dichloro-1,1,2,2,3-pentafluoropropane (HCFC-225cb) and so on can be used.
As the solvent (also serving as an extraction distillation solvent), CH₂Cl₂, CHCl₃, CCl₄, CH₂ClCH₂Cl, toluene, diisopropylether and so on can also be used.

Thus, hexafluoropropylene oxide is produced by the above procedures. This process for producing hexafluoropropylene oxide can be continuously performed.

According to the present embodiment, the yield of HFPO can be remarkably improved in comparison with the conventional processes.

### Examples

Examples of the present invention are described in detail below with reference to the accompanying drawings.

### (Example 1)

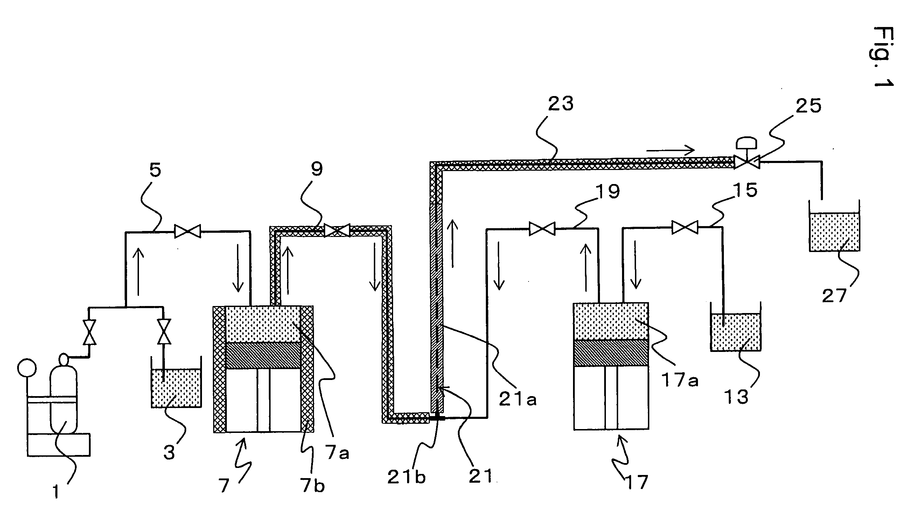

Referring to Fig. 1, this Example relates to an example which uses an internal space of a capillary 21 (indicated by the dotted line in the drawing) as the small space. As the capillary 21, a SUS tube having a nominal inner diameter of 250 µm and a length of 1.5 m was used. The capillary 21 was capable of controlling a temperature by heating using a heating jacket 21a. The inlet end of this capillary 21 was connected to a T type SUS connector 21b (adaptable outer diameter of 1/16 inch, from Swagelok Company), thus making it possible to simultaneously feed two kinds of fluids of an organic phase and an aqueous phase to the capillary 21 through lines 9 and 19. The outlet side of the capillary 21 was connected to a line 23. As the line 23, a SUS tube having a nominal inner diameter of 500 µm was used. At the connections, appropriate members such as nuts were used.

Firstly, HFP was drawn from a HFP tank 1 and an organic solvent was drawn from an organic solvent bath 3 into a pump room 7a of a syringe pump 7 through a line 5 as shown in Fig. 1. As the organic solvent, 1,1-dichloro-1-fluoroethane (HCFC-141b) was used. Tri-n-octylmethylammonium chloride (TOMAC: (C₈H₁₇)₃CH₃NCl) as a phase transfer catalyst was preliminarily dissolved in the organic solvent at about 6% by weight. In the pump room 7a, a mixture of HFP and the organic solvent (containing the phase transfer catalyst) was cooled to about -5°C by a cooling jacket 7b which surrounds the pump room 7a. This mixture was extruded from the syringe room 7a and supplied as an organic phase to the capillary 21 through a line 9. The line 9 was also cooled to about -5°C from surroundings (in the drawing, a cooling portion around the line 9 is indicated by hatching).

The organic phase was at about -5°C under about 2 MPa when it was supplied to the capillary 21. At that time, substantially the entire HFP was liquefied and the concentration of HFP in the organic phase was about 1.1% by weight. The concentration of TOMAC (the phase transfer catalyst) in the organic phase was the substantially the same as that in the organic solvent used.

On the other hand, an aqueous solution was drawn from an aqueous solution bath 13 into a pump room 17a of a syringe pump 17 through a line 15. This aqueous solution was prepared by dissolving about 10% by weight of sodium hypochlorite (NaClO) as an oxygen-containing oxidizing agent in water. This aqueous solution was extruded from the syringe room 17a and supplied as an aqueous phase to the capillary 21 through a line 19.

The aqueous phase was at about room temperature (about 20°C) under about 2 MPa when it was supplied to the capillary 21. The concentration of NaClO (the oxygen-containing oxidizing agent) in the aqueous phase was the same as that in the aqueous solution used.

The supply flow rate of the organic phase was about 1 mL/min, and the supply flow rate of the aqueous phase was about 250 µL/min.

The organic phase and the aqueous phase supplied to the capillary 21 flowed through the small space inside the capillary 21 in the presence of the catalyst while contacting with each other in a state of laminar flow. At that time, the capillary 21 was heated to about 45°C by the heating jacket 21a and the pressure was adjusted by a back pressure valve 25 existing in a downstream line 23. Thus, the inside of the capillary 21 was maintained at about 45°C under about 2 MPa.

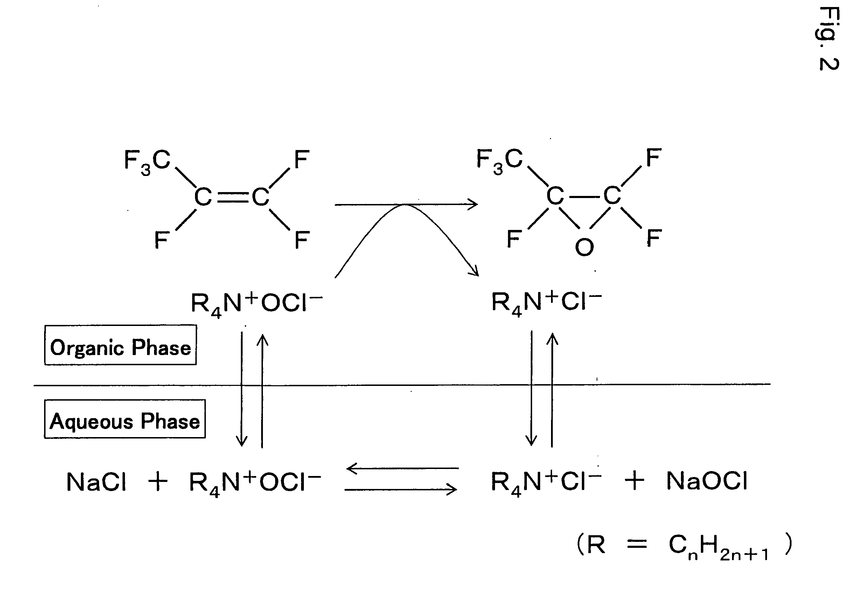

In the small space inside the capillary 21, HFP was reacted with NaClO by a catalyst action of TOMAC to give HFPO. While the present invention is not intended to be bound by any specific theory, an expected reaction scheme is shown in Fig. 2 (in the drawing, three alkyl groups of the four alkyl groups have n=8 and the remaining one group has n=1 in this Example). Further, it was confirmed that carbon dioxide (CO₂) existed in a vapor phase and trifluoroacetic acid (CF₃COOH) existed in the aqueous phase, which were the main products from a side reaction(s).

Referring to Fig. 1, the reaction mixture (a mixture of the organic phase and the aqueous phase after the reaction) was drawn from the capillary 21 into a recovery bath 27 through a line 23. The line 23 was maintained at about 0°C by an ice bath (in the drawing, a cooling portion around the line 23 is indicated by hatching).

The residence time of the fluid (comprising the organic phase and the aqueous phase, and the vapor phase if present) in the capillary 21 was about 1.1 seconds. Since the line 23 was maintained at a low temperature of about 0°C as described above, it can be considered that the reaction does not substantially occur in the line 23. Thus, the residence time of the fluid in the capillary 21 can be considered to be the reaction time.

The recovered reaction mixture was left to separate into the organic phase and the aqueous phase. The resulting organic phase was analyzed by gas chromatography. The results showed that a conversion rate of HFP was 99% and a selectivity of HFPO was about 94%. Thus, yield was about 92%.

### (Examples 2-4)

An apparatus which was same as in Example 1 was used, except that a SUS tube having a nominal inner diameter of 500 µm and a length of 4 m was used as the capillary 21. Procedures which were same as in Example 1 were conducted, except that a mixture containing components shown in Table 1 was used as an organic phase, the organic phase was supplied at each supply flow rate shown in Table 1, and the aqueous phase was supplied at a supply flow rate of about 24 ml/min while the used aqueous phase was an aqueous sodium hypochlorite solution at about 10% by weight as in Example 1. Thus resulting organic phase was analyzed by gas chromatography. Results are shown in Table 2.

**Table 1**

| Example | Organic phase | | | |
|---|---|---|---|---|
| | Components | | | Supply flow rate (ml/min) |
| | Solvent | Phase transfer catalyst | HFP | |
| 2 | None | None | HFP 7 g | 0.5 |
| 3 | Cyclohexane 72 ml | TOMAC 0.48 g | HFP 7 g | 0.5 |
| 4 | HFC-365 72 ml | TOMAC 0.48 g | HFP 7 g | 0.1 |

**Table 2**

| Example | Conversion rate (%) | Selectivity (%) | Yield (%) |
|---|---|---|---|
| 2 | 6 | 99 | 6 |
| 3 | 98 | 99 | 98 |
| 4 | 22 | 99 | 22 |

### (Example 5)

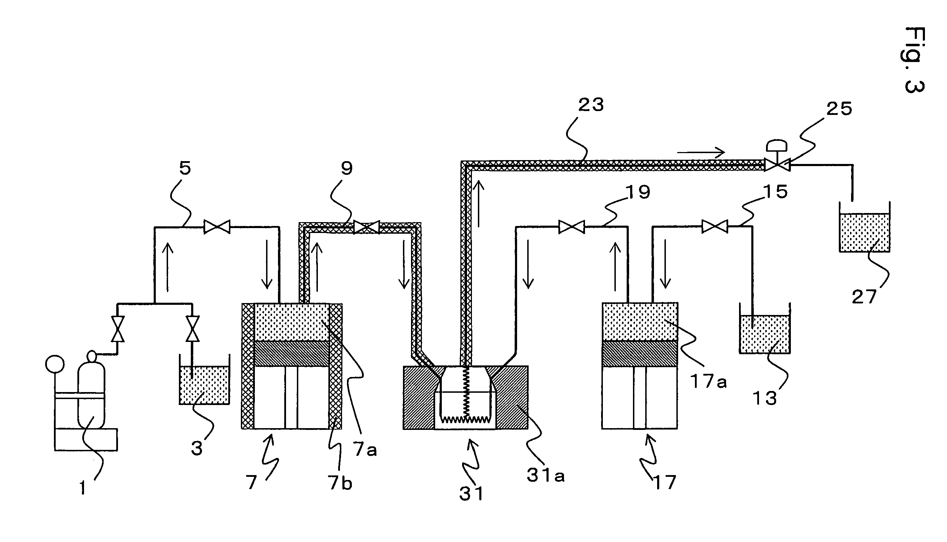

Referring to Fig. 3, this Example relates to an example which uses unit spaces in a micromixer 31 as the small space. As the micromixer 31, SSIMM (Standard Slit Interdigital Micro Mixer from IMM, nominal slit width of 40 µm) was used. In this Example, tetrabutylammonium hydrogen sulfate (TBAS) was used as a phase transfer catalyst in place of TOMAC. Others components are the same as in Example 1 unless otherwise specified.

An organic phase and an aqueous phase were supplied to the micromixer 31. The micromixer 31 was constructed as follows. The micromixer 31 was provided with plural slits (nominal width of 40 µm) at its lower inside. The slits were isolated from each other by wavy vertical walls and closed at right and left ends alternately. The aqueous phase and the organic phase were supplied to the plural slits alternately and oppositely from the right and left directions (in the form of stripes), and then flowed upward in a vertical direction between the slits. After passing the upper end of the slits, the aqueous phase and the organic phase were contacted with each other in a state of laminar flow, and then taken out from the micromixer 31 in a mixed state. That is, the organic phase and the aqueous phase flowed in the form of alternate multiple layers, and one pair of a layer of the organic phase and a layer of the aqueous phase was a unit space, this unit space was the small space. Therefore, the organic phase and the aqueous phase supplied to the micromixer 31 flowed through the plural small spaces inside the micromixer 31 in the presence of the catalyst while contacting with each other in a state of laminar flow. At that time, the micromixer 31 was heated to about 35°C by the heating jacket 31a and the pressure was adjusted as in Example 1 by a back pressure valve 25 existing in a downstream line 23. Thus, the inside of the micromixer 31 was maintained at about 35°C under about 2 MPa.

In each of the small spaces inside the micromixer 31, HFP was reacted with NaClO by a catalyst action of TBAS to give HFPO.

The reaction mixture (a mixture of an organic phase and an aqueous phase after the reaction) was drawn from the micromixer 31 into a recovery bath 27 through a line 23. The residence time of the fluid (comprising the organic phase and the aqueous phase, and the vapor phase if present) in the micromixer 31 was 1 second or less. Thus, the reaction time may be considered to be 1 second or less.

The recovered reaction mixture was left to separate into the organic phase and the aqueous phase. The resulting organic phase was analyzed by gas chromatography. The results showed that a conversion rate of HFP was about 100% and selectivity of HFPO was about 85%. Thus, yield was about 85%.

While various Examples of the present invention have been described, the apparatuses used in these Examples were merely applied for carrying out in the laboratory scale, and a method for preparation of an organic phase and an aqueous phase, a method for supplying them, and a method for control of the temperature and pressure can be modified appropriately.

### (Comparative Example 1)

This Comparative Example related to an example where the reaction was conducted by a batch-wise manner in a reactor of a milliliter order volume, without using a small space. As the reactor, a pressure-resistant vessel having a volume of 200 mL was used.

Firstly, 72 mL of an organic solvent in which 0.48 g of TBAS was preliminarily dissolved in HCFC-141b was charged in a reactor, followed by sealing. Air in the reactor was replaced with nitrogen, and the reactor was situated in vacuum state. Then, 5.0 g of HFP was added thereto to prepare an organic phase. This reactor was disposed in a constant temperature bath at 0C°, and its content was stirred by a stirrer until the temperature of the organic phase reached an equilibrium state. On the other hand, NaClO was dissolved in water at about 10% by weight to prepare an aqueous solution as an aqueous phase. This aqueous phase was added to the organic phase in the reactor at a rate of 2 mL/min.

The gas phase obtained at predetermined elapsed time points since the beginning of addition was analyzed by gas chromatography. The results showed as follows. For the elapsed time of 30 minutes, selectivity was about 97% and a conversion rate was about 41%, and thus yield was about 40%. For the elapsed time of 60 minutes, selectivity was about 97% and a conversion rate was about 55%, and thus yield was about 53%. For the elapsed time of 120 minutes, selectivity was about 59% and a conversion rate was about 88%, and thus the yield was about 52%.

### (Comparative Example 2)

Procedures which were same as in Comparative Example 1 were conducted, except that only HFP was used as an organic phase (that is, a solvent and a phase transfer catalyst were not used). The gas phase obtained at predetermined elapsed time points since the beginning of addition was analyzed by gas chromatography. The results showed that, in the all cases of the elapsed times of 30 minutes, 60 minutes and 120 minutes, a conversion rate was 0% and proceeding of the reaction was not observed, and thus the yield was 0%.

### Industrial Applicability

Hexafluoropropylene oxide produced by the process of the present invention can be used in the production of fluorine-containing compounds such as perfluorovinylether, and also can be used as a lubricating oil or a heating medium in the form of an oligomer.

## Claims

1. A process for producing hexafluoropropylene oxide, which comprises passing an organic phase comprising hexafluoropropylene and an aqueous phase comprising an oxygen-containing oxidizing agent through a small space in contact with each other, thereby reacting hexafluoropropylene with the oxygen-containing oxidizing agent to obtain hexafluoropropylene oxide.

2. The process according to claim 1, wherein the small space has a passage width of 3 cm or less.

3. The process according to claim 1 or 2, wherein the organic phase comprising hexafluoropropylene and the aqueous phase comprising an oxygen-containing oxidizing agent are contacted with each other in the presence of a phase transfer catalyst, thereby reacting hexafluoropropylene with the oxygen-containing oxidizing agent by an action of the phase transfer catalyst.

4. The process according to any one of claims 1 to 3, wherein the small space is maintained at -40 to 100°C and 0.1 to 20 MPa.

5. The process according to any one of claims 1 to 4, wherein the organic phase is subjected to conditions of -40 to 100°C and 0.1 to 20 MPa before the organic phase is supplied to the small space.

6. The process according to claim 5, wherein the organic phase is subjected to temperature and pressure conditions at which hexafluoropropylene is substantially in a liquid state before the organic phase is supplied to the small space.

7. The process according to any one of claims 1 to 6, wherein a reaction time in the small space is from 0.01 to 1,000 seconds.

8. The process according to claim 3, wherein the phase transfer catalyst is a quaternary ammonium salt.

9. The process according to any one of claims 1 to 8, wherein the oxygen-containing oxidizing agent is a hypochlorite.
